# EUROPÄISCHE PATENTSCHRIFT

(11) **EP 1 648 858 B1**
(45) Veröffentlichungstag und Bekanntmachung des Hinweises auf die Patenterteilung: **02.07.2008**
(21) Anmeldenummer: 04738049.8
(22) Anmeldetag: 29.06.2004
(51) Int. Cl.: C07C 231/12, C07D 207/38, C07C 233/09

(54) **VERFAHREN ZUR HERSTELLUNG VON ALPHA, BETA-UNGESATTIGTEN AMI DVERBINDUNGEN**
METHOD FOR PRODUCTION OF ALPHA, BETA-UNSATURATED AMIDE COMPOUNDS
PROCEDE DE PRODUCTION DE COMPOSES AMIDE ALPHA, BETA-INSATURES

(30) Priorität: 21.07.2003 CH 127403
(43) Veröffentlichungstag der Anmeldung: 26.04.2006
(73) Patentinhaber: Siegfried Generics International AG, 4800 Zofingen (CH)
(72) Erfinder: WEBER, Beat, CH-4800 Zofingen (CH); SCHÄRER, Norbert, CH-5036 Oberentfelden (CH); MÜLLER, Beat-W., CH-4106 Therwil (CH)
(74) Vertreter: Braun, André jr.
(86) Internationale Anmeldenummer: PCT/CH2004/000408
(87) Internationale Veröffentlichungsnummer: WO 2005/007618

(56) Entgegenhaltungen:
- EP-A- 0 298 652
- EP-A- 0 428 366
- EP-A- 0 473 226
- BHATTACHARYA APURBA ET AL: "Silylation-mediated oxidation of 4-aza-3-ketosteroids with DDQ proceeds via DDQ-substrate adducts" JOURNAL OF THE AMERICAN CHEMICAL SOCIETY, AMERICAN CHEMICAL SOCIETY, WASHINGTON, DC, US, Bd. 110, 1988, Seiten 3318-3319, XP002179347 ISSN: 0002-7863
- BROWN H.C. ET AL.: "SELECTIVE REDUCTIONS. V. THE PARTIAL REDUCTION OF TERTIARY AMIDES BY LITHIUM DI- AND TRIETHOXYALUMINOHYDRIDES - A NEW ALDEHYDE SYNTHESIS VIA THE DIMETHYLAMIDES" J. AM. CHEM. SOC., Bd. 86, 1964, Seiten 1089-1095, XP002262813
- BAKER J.T. ET AL.: "SYNTHESIS AND PROPERTIES OF PYRROLIN-2-ONES" J. ORG. CHEM., Bd. 44, Nr. 15, 1979, Seiten 2798-2800, XP002262814
- CARAMELLA P ET AL: "Cycloaddition of Nitrile Oxides to Cyclic and Acyclic alpha,beta-Unsaturated Amides. Frontier Orbital Interactions and an Unexpected Steric Drift Determine Regiochemistry" TETRAHEDRON, ELSEVIER SCIENCE PUBLISHERS, AMSTERDAM, NL, Bd. 55, Nr. 22, 28. Mai 1999 (1999-05-28), Seiten 7027-7044, XP004165609 ISSN: 0040-4020
- HORI K ET AL: "cis-Selective Aziridination of cis- or trans-alpha,beta-Unsaturated Amides Using Diaziridine" TETRAHEDRON LETTERS, ELSEVIER SCIENCE PUBLISHERS, AMSTERDAM, NL, Bd. 40, Nr. 28, 9. Juli 1999 (1999-07-09), Seiten 5207-5210, XP004170055 ISSN: 0040-4039
- KANEMASA S ET AL: "dl-Selective Reductive Coupling/Dieckmann Condensation Sequence of alpha,beta-Unsaturated Amides with Samarium(II) Iodide/HMPA. Synthesis of a New Ligand, trans-1,2-Cyclopentanediyl-2,2 ?,-biphenol " TETRAHEDRON LETTERS, ELSEVIER SCIENCE PUBLISHERS, AMSTERDAM, NL, Bd. 37, Nr. 47, 18. November 1996 (1996-11-18), Seiten 8505-8506, XP004068701 ISSN: 0040-4039
- TSUJI J ET AL: "PALLADIUM CATALYZED PREPARATION OR lpha.-ALLYL ETSRS AND alpha,beta-UNSATURATED ESTERS FROM SATURATED ESTERS VIA THEIR KETENE SILYL ACETALS" TETRAHEDRON LETTERS, ELSEVIER SCIENCE PUBLISHERS, AMSTERDAM, NL, Bd. 25, Nr. 42, 1984, Seiten 4783-4786, XP002226639 ISSN: 0040-4039 in der Anmeldung erwähnt

## Beschreibung

Die vorliegende Erfindung betrifft Verfahren zur Herstellung von α,β-ungesättigte Amidverbindungen bzw. Verfahren zur Einführung einer α,β-ungesättigten Doppelbindung in Verbindungen, welche eine Amidgruppierung enthalten, indem man die entsprechende gesättigte Amidverbindung in der α,β-Stellung dehydriert.

EP 298 652 beschreibt die Dehydrogenierung von 3-Oxo-4-azasteroiden durch Reaktion mit einem Silylierungsmittel in Gegenwart eines Chinons.

Die vorliegende Erfindung betrifft Verfahren zur Herstellung von α,β-ungesättigten Amidverbindungen der allgemeinen Formel (I): 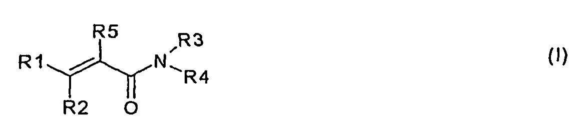 worin
R₁ und R₂ unabhängig voneinander Wasserstoff; gegebenenfalls mit Hydroxy, Halogen, Phenyl, substituiertem Phenyl, oder mit einer Estergruppe [-C(O)OAlkyl] oder einer Amidgruppe [-C(O)NH₂ oder -C(O)NHAlkyl], substituiertes lineares oder verzweigtes (C₁-C₁₈)-Alkyl oder (C₁-C₁₈)-Alkenyl; gegebenenfalls mit Halogen substituiertes Phenyl; oder
   entweder R₁ oder R₂ einen Rest Y-R_{6;} worin
Y Sauerstoff (-O-) ; Schwefel (-S-); -NR₇-; oder Dialkylsilyloxy [-(Alkyl)₂Si-O-];
R₆ Wasserstoff, gegebenenfalls mit Hydroxy, Halogen, Phenyl, substituiertem Phenyl oder mit einer Estergruppe [-C(O)OAlkyl] oder einer Amidgruppe [-C(O)NH₂] oder [-C(O)NHAlkyl], substituiertes lineares oder verzweigtes (C₁-C₁₈)-Alkyl; gegebenenfalls mit Halogen substituiertes Phenyl;
R₇ (C₁-C₁₈)-Alkyl oder -N(R₆)(R₇) einen 5- oder 6-gliedrigen heterocyclischen Ring; oder
R₁ zusammen mit R₃ eine direkte Bindung oder einen Rest der Formel -(CH₂)ₙ-; worin
n eine ganze Zahl von 1 bis 12; oder
R₁ zusammen mit R₂ Cyclohexyliden; oder
R₁ zusammen mit R₅ und der eingeschlossenen (C=C)-Doppelbindung Cyclohexenyl; oder
R₁ zusammen mit R₅ und der eingeschlossenen (C=C)-Doppelbindung einen Rest eines einfach-ungesättigten Bicyclischen Ringes;
R₃ Wasserstoff, gegebenenfalls durch Phenyl, Hydroxyl, oder Halogen substituiertes lineares oder verzweigtes (C₁-C₁₂)-Alkyl, welches gegebenenfalls durch ein oder mehrere Sauerstoffatome unterbrochen ist, (C₅-C₈)-Cycloalkyl oder (C₅-C₈)-Cycloalkenyl, welche gegebenenfalls durch ein oder mehrere Sauerstoffatome unterbrochen sind; gegebenenfalls durch Halogen oder Hydroxyl substituiertes Phenyl; oder R₃ zusammen mit R₁ eine direkte Bindung oder einen Rest der Formel -(CH₂)ₙ-;
R₄ eine der Bedeutungen von R₃, vorzugsweise Wasserstoff, gegebenenfalls durch Phenyl, Hydroxyl, oder Halogen substituiertes lineares oder verzweigtes (C₁-C₁₂)-Alkyl, gegebenenfalls durch Halogen oder Hydroxyl substituiertes Phenyl; oder -NR₃R₄ einen 5- oder 6-gliedrigen heterocyclischen Ring; bedeuten und
R₅ eine der für R₁ oder R₂ als unabhängige Substituenten angegebenen Bedeutungen hat [d.h. Wasserstoff; gegebenenfalls mit Hydroxy, Halogen, Phenyl, substituiertem Phenyl, oder mit einer Estergruppe [-C(O)OAlkyl] oder einer Amidgruppe [-C(O)NH₂ oder -C(O)NHAlkyl], substituiertes lineares oder verzweigtes (C₁-C₁₈)-Alkyl oder (C₁-C₁₈) -Alkenyl; oder gegebenenfalls mit Halogen substituiertes Phenyl]; welches dadurch gekennzeichnet ist, dass man
   (A) in einer Verbindung der allgemeinen Formel (II): 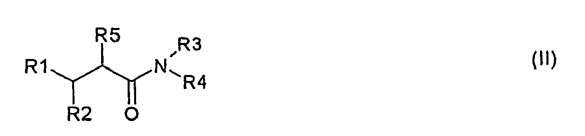 worin R₁, R₂, R₃, R₄ und R₅ entsprechend die oben angegebenen Bedeutungen haben und [die Verbindung der Formel (II)] die entsprechende gesättigte Verbindung darstellt, Schutzgruppen einführt, so dass eine Verbindung der allgemeinen Formel (III) entsteht: 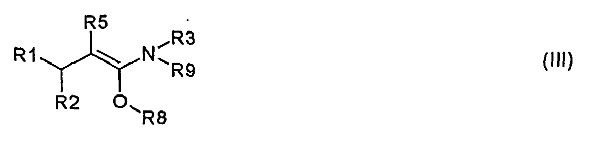 worin
      - R₈: Trialkylsilyl, oder (wenn R₄ = Wasserstoff) zusammen mit R₉ den Rest -C(O)-(CH₂)ₘ-C(O)- und
      - R₉: (wenn R₄ = Wasserstoff) Alkyloxycarbonyl oder Phenyloxycarbonyl, vorzugsweise Boc (= tert.-Butyloxycarbonyl); oder Trialkylsilyl, oder zusammen mit R₈ den Rest -C(O)-(CH₂)ₘC(O)-, und
      - m: 0, 1, 2, oder 3, vorzugsweise 0 oder 1, vorzugsweise 0, bedeuten,
      und worin für den Fall, dass in der Verbindung der allgemeinen Formel (II) Hydroxyl anwesend ist, dieses gegebenenfalls mit einer monovalenten Schutzgruppe R₈ und/oder R₉ reagiert hat;
   (B) die [gemäss Schritt (A)] erhaltene Verbindung in Gegenwart (i) eines Dehydrierungskatalysators ausgewählt aus Verbindungen der Gruppe der Übergangsmetalle des Periodensystems der Elemente, vorzugsweise aus Verbindungen der Metalle der VIII. Gruppe des Periodensystems, und in Gegenwart (ii) eines Oxidationsmittels ausgewählt aus der Gruppe enthaltend gegebenenfalls substituiertes Benzochinon, Allylmethylcarbonat, Allylethylcarbonat und/oder Allylpropylcarbonat, umsetzt, wobei die α,β-Doppelbindung in die α,β-Stellung eingeführt wird, und
   (C) gegebenenfalls anwesende Schutzgruppen R₈, sowie den Substituent R₉ entfernt.

Als Oxidationsmittel [in Schritt (B)] sind sowohl organische wie auch anorganische Verbindungen geeignet, welche aus Palladium-Verbindungen der Oxidationsstufe Null Palladium-Verbindungen der Oxidationsstufe +II ergeben. Beispielsweise reagiert Allyl-methyl-carbonat wie aus der Literatur bekannt (Tetrahedron Letters, Vol 25., No 42, 4783-4786, 1984) durch oxidative Addition an Palladium(0) zu den entsprechenden Palladium(II) Allyl-Derivaten. Weitere derart wirkende Oxidationsmittel sind dem Fachmann bekannt. Zu erwähnen ist, das in Schritt (B) gleichzeitig der über Sauerstoff an die Amideinheit gebundene Substituent R₈ entfernt wird.

R₁ und R₂ bedeuten unabhängig voneinander vorzugsweise Wasserstoff; gegebenenfalls mit Hydroxy, Phenyl, mit Halogen oder Hydroxy substituiertem Phenyl, oder mit einer (C₁₋₄)Alkyl-Estergruppe [-C(O)O(C₁₋₄)Alkyl] oder einer Amidgruppe [-C(O)NH₂] oder (C₁₋₄)Alkyl-Amidgruppe [-C(O)NH(C₁₋₄)Alkyl] substituiertes lineares oder verzweigtes (C₁-C₈)-Alkyl oder (C₁-C₈)-Alkenyl; gegebenenfalls mit Halogen substituiertes Phenyl; vorzugsweise lineares oder verzweigtes (C₁-C₈)-Alkyl oder (C₁-C₈)-Alkenyl; Benzyl oder Phenyl.

Vorzugsweise bedeutet R₂ Wasserstoff und R₁ vorzugsweise lineares oder verzweigtes (C₁-C_{B}) -Alkyl oder (C₁-C₈)-Alkenyl; Benzyl oder Phenyl oder Y-R₆, wobei für Y-R₆ die im weiteren gegebenen Definitionen und Einschränkungen gelten oder R₁ bedeutet Wasserstoff und R₂ hat die breite (vorgehend für R₁ angegebene) Bedeutung.

Bevorzugt sind auch die Bedeutungen, worin R₁ zusammen mit R₃ die direkte Bindung oder einen Rest der Formel -(CH₂)ₙ- und n eine ganze Zahl von 1 bis 12; oder R₁ zusammen mit R₂ Cyclohexyliden; oder R₁ zusammen mit R₅ Cyclohexenyl, bedeuten.

Bedeutet entweder R₁ oder R₂ einen Rest Y-R₆, so bedeutet darin Y vorzugsweise Sauerstoff (-O-).

Bedeuten R₁ zusammen mit R₃ eine direkte Bindung oder einen Rest der Formel -(CH₂)ₙ-, so bedeutet die Verbindung der Formel (I) vorzugsweise ein Lactam einer omega-Amino-Fettsäure, beispielsweise der omega-Aminobuttersäure (ω-Butyrolactam), der omega-Aminovaleriansäure (ω-Valerolactam), der omega-Aminocapronsäure (ω-Caprolactam), oder der omega-Aminolaurinsäure (ω-Laurinolactam), welche gemäss der Verbindung der allgemeinen Formel (I) eine α,β-ungesättigte Doppelbindung aufweisen.

Bedeutet R₁ zusammen mit R₅ und der eingeschlossenen (C=C)-Doppelbindung einen Rest eines einfach ungesättigten Bicyclischen Ringes, so ist dies vorzugsweise ein gegebenenfalls durch Hydroxyl oder Amino substituierter Norbornylrest, vorzugsweise einen Norbornylrest.

R₃ bedeutet vorzugsweise Wasserstoff, gegebenenfalls durch Phenyl substituiertes lineares oder verzweigtes (C₁-C₄)-Alkyl; Cyclohexyl; Phenyl; oder R₃ zusammen mit R₁ eine direkte Bindung oder einen Rest der Formel -(CH₂)ₙ-.

R₄ bedeutet vorzugsweise Wasserstoff, gegebenenfalls durch Phenyl substituiertes lineares oder verzweigtes (C₁-C₄)-Alkyl oder Phenyl, vorzugsweise Wasserstoff.

Der Rest -NR₃R₄ als heterocyclischer Ring bedeutet vorzugsweise einen Rest von Pyrrolidin oder Piperidin.

R₅ bedeutet vorzugsweise Wasserstoff, tert.-Butyl oder gegebenenfalls durch Halogen oder Hydroxyl substituiertes Phenyl, vorzugsweise Wasserstoff.

R₆ bedeutet vorzugsweise Wasserstoff, gegebenenfalls mit Hydroxy, Halogen, Phenyl, mit Halogen substituiertem Phenyl, oder mit einer (C₁₋₄)Alkyl-Estergruppe [-C(O)O(C₁₋₄)-Alkyl] oder einer Amidgruppe [-C(O)NH₂] oder einer (C₁₋₄)Alkyl-Amidgruppe [-C(O)NH(C₁₋₄)Alkyl] substituiertes lineares oder verzweigtes (C₁-C₈)-Alkyl; gegebenenfalls mit Halogen substituiertes Phenyl; vorzugsweise Wasserstoff, gegebenenfalls mit Phenyl, oder mit einer (C₁₋₄)-Alkyl-Estergruppe oder einer Amidgruppe oder einer (C₁₋₄)-Alkyl-Amidgruppe substituiertes lineares oder verzweigtes (C₁-C₈)-Alkyl; oder Phenyl; vorzugsweise Wasserstoff, lineares oder verzweigtes (C₁-C₈)-Alkyl oder Phenyl.

R₇ bedeutet vorzugsweise (C₁-C₄)-Alkyl. Der Substituent -N(R₆)(R₇) als heterocyclischen Ring bedeutet vorzugsweise einen Rest von Pyrrolidin oder Piperidin.

R₈ bedeutet vorzugsweise Trimethylsilyl, oder zusammen mit R₉ den Rest -C(O)-(CH₂)ₘ-C(O)-, worin m 0, 1, 2, oder 3, vorzugsweise 0 oder 1, vorzugsweise Null, bedeutet.

R₉ bedeutet vorzugsweise Boc, Trimethylsilyl, oder zusammen mit R₈ den Rest -C(O)-(CH₂)ₘ-C(O)-, worin m 0, 1, 2, oder 3, vorzugsweise 0 oder 1, vorzugsweise Null, bedeutet. Vorzugsweise bedeutet R₉ Boc oder zusammen mit R₈ den Rest -C(O)-(CH₂)ₘ-C(O)-, worin m vorgehend angegebenen Bedeutungen hat, vorzugsweise Boc oder zusammen mit R₈ den Rest -C(O)-C(O)-.

R₉ als Alkyloxycarbonyl bedeutet vorzugsweise Isobutyloxycarbonyl, tert.-Butyloxycarbonyl, tert.-Amyloxycarbonyl, Cyclobutyloxycarbonyl, 1-Methylcylobutyloxycarbonyl, Cyclopentyloxycarbonyl, Cyclohexyloxycarbonyl, 1-Methylcyclohexyl, vorzugsweise tert.-Butyloxycarbonyl.

Dialkylsilyl bedeutet vorzugsweise Dimethylsilyl. Trialkylsilyl bedeutet vorzugsweise Trimethylsilyl. Halogen bedeutet vorzugsweise Fluor oder Chlor, vorzugsweise Fluor. Eine Alkyl-Estergruppe bedeutet vorzugsweise eine Methyl-, Ethyl-, Propyl- oder Butylestergruppe. Eine Alkylamidgruppe bedeutet vorzugsweise eine Methyl-, Ethyl-, Propyl- oder Butylamidgruppe.

Verbindungen, welche erfindungsgemäss hergestellt und unter die allgemeine Formel (I) subsumiert werden können sind beispielsweise die entsprechenden α,β-ungesättigten Verbindungen von N,N-Dialkyl-alkylamiden, wie z.B. N,N-Dimethylbutylamid und homologen Verbindungen, oder von den vorgehend erwähnten Lactamen. Weitere Beispiel für die erfindungsgemässe Herstellung sind: 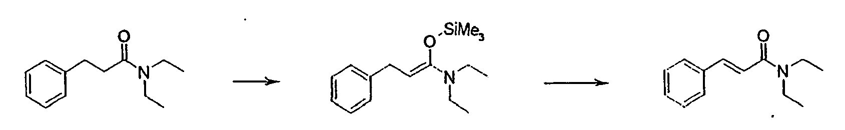 oder

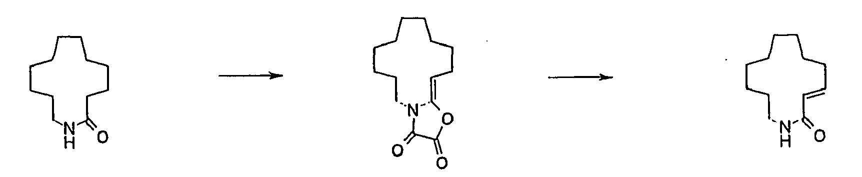

Zur Einführung der Schutzgruppe Trialkylsilyl, d.i. zur Silylierung der NH-Gruppe und/oder des Sauerstoffatoms bzw. der OH-Gruppe [gemäss Schritt (A)], verwendet man vorzugsweise ein (Alkyl)₃Si(Halogen), z.B. (CH₃)₃SiCl, oder Bistrimethylsilyltrihalogenacetamid, Bistrimethylsilylacetamid, Hexamethyldisilazan und/oder Bistrimethylharnstoff, vorzugsweise Bistrimethylsilyltrifluoroacetamid, oder ein Trialkylsilyl-trifluoromethansulfonat, vorzugsweise Trimethylsilyl-trifluoromethansulfonat. Die Reaktionsbedingungen für die Silylierung sind aus EP 0 473 226 bekannt.

Für die Einführung einer Schutzgruppe, worin R₇ zusammen mit R₈ den Rest -C(O)-(CH₂)ₘ-C(O)- bedeutet und worin m die vorgehend angegebenen Bedeutungen hat, setzt man die Verbindung der allgemeinen Formel (II) bzw, die Laktamgruppierung [gemäss Schritt (A)] mit dem entsprechenden Dihalogenid, vorzugsweise Oxalylchlorid (Oxalsäurechlorid) oder Malonylchlorid (Malonsäurechlorid) um, wobei Oxalylchlorid bevorzugt ist. Die Reaktionsbedingungen für die Umsetzung mit Oxalylchorid sind EP 0 428 366 bekannt und sind für die Umsetzung mit Malonylchlorid oder analog reagierender Verbindungen in analoger Weise anzuwenden.

Für die Einführung einer Schutzgruppe, worin R₈ Alkyloxycarbonyl, z.B. tert.-Butyloxycarbonyl (Boc) bedeutet, geht man in an sich bekannter Weise vor, indem man die Verbindung der allgemeinen Formel (II) z.B. mit Boc-Anhydrid (Boc-O-Boc) {[(CH₃)₃C-O-C(O)]₂-O} oder mit Boc-Carbamat [(CH₃)₃C-O-C(O)-N(C₁₋₄-Alkyl)₂], umsetzt. Dabei steht hier Boc stellvertretend für die anderen gleich reagierenden Verbindungen, das heisst Verbindungen, worin der tert.-Butylrest ersetzt ist durch einen andern gleich reagierenden Rest, wie beispielsweise die genannten Reste tert.-Amyl, Cyclobutyl, Cyclopentyl oder Cyclohexyl. Solche analogen Reaktionen sind zahlreich in der Fachliteratur beschrieben. Bedeutet R₈ Trialklylsilyl und R₉ Boc, so führt man zuerst die Schutzgruppe Boc ein und silyliert anschliessend.

In Schritt (B) wird die gemäss Schritt (A) erhaltene Verbindung in Gegenwart (i) eines Dehydrierungskatalysators und in Gegenwart (ii) eines geeigneten Oxidationsmittels, wie gegebenenfalls substituiertem Benzochinon, Allylmethylcarbonat, Allylethylcarbonat und/oder Allylpropylcarbonat, umgesetzt, wobei die α,β-Doppelbindung in α,β-Stellung eingeführt wird. Der Dehydrierungskatalysator ist ausgewählt aus Verbindungen (Salze und Komplexe) der Gruppe der Übergangsmetalle des Periodensystems der Elemente, insbesondere ausgewählt aus Verbindungen der Metalle der VIII. Gruppe des Periodensystems, insbesondere von Eisen (Fe), Ruthenium (Ru) und Osmium (Os); Cobalt (Co), Rhodium (Rh), und Iridium (Ir); Nickel (Ni), Palladium (Pd) und Platin (Pt) sowie der Gruppe IB, d.h. von Kupfer (Cu), Silber (Ag) und Gold (Au). Bevorzugt sind Verbindungen der Metalle der Gruppe VIII des Periodensystems. Bevorzugt sind insbesondere Verbindungen bzw. Dehydrierungskatalysatoren auf der Basis von Rhodium (Rh), Palladium (Pd) und Platin (Pt). Bevorzugt sind Palladiumverbindungen. Beispiele für solche Palladiumverbindungen sind: Pd(0)-Verbindungen wie Tris(dibenzylidenaceton)-diPalladium-ChloroformKomplex und Pd(II)-Verbindungen wie PdCl₂, Pd(dppe)₂, [dppe = bis-(1,2-biphenylphosphino)-ethan], Pd(dppe)Cl₂, Pd(OAc)₂, Pd (dppe) (OAc)₂, π-Allyl-Pd-Komplexe, vorzugsweise π-Allyl-Pd-chlorid Dimer. Bevorzugt sind Pd(0)-Verbindungen, insbesondere Tris(dibenzylidenaceton)diPalladium ChloroformKomplex. Diese Verbindungen, bzw. Salze und Komplexe, sind an sich bekannt und in der Literatur beschrieben worden.

Zur termischen Stabilisierung des Palladium-Komplexes kann ein zusätzlicher Komplexbildner wie 2,2'-Bipyridyl oder 1,10-Phenanthrolin eingesetzt werden, vorzugsweise 2,2'-Bipyridyl.

Als Chinon kann man auch ein substituiertes Chinon verwenden, beispielsweise ein durch C₁₋₄-Alkyl, Halogen, Cyano oder Nitro substituiertes Chinon. Solche Chinone sind an sich bekannt.

Erklärungshalber kann zum Mechanismus der Katalyse angeführt werden, dass eine Pd-Spezies am C-Atom in 2-Stellung unter Abspaltung der Sauerstoff-Schutzgruppe [z.B. der -Si(CH₃)₃-Gruppe] addiert. Eine anschliessende beta-Wasserstoff-Abspaltung am C-Atom in 1-Stellung führt zur gewünschten Δ¹-Doppelbindung in 1-/2-Stellung, und setzt eine weitere Palladium-Spezies frei, die in den katalytischen Zyklus zurück geführt wird. Hinweise für diesen Reaktionsmechanismus finden sich in Tetrahydron Letters, Seite 4783, (1984). Die vorliegende Erfindung ist aber nicht an diese Erklärung gebunden.

In Schritt (C) wird dann die erhaltene Verbindung in die Verbindung der Formel (I) umgewandelt, indem man die eingeführten Schutzgruppen entfernt. Dies geschieht vorzugsweise durch Behandlung mit einer geeigneten Säure, beispielsweise mit Ameisensäure, Essigsäure und/oder Trifluoressigsäure, vorzugsweise mit Ameisensäure. Methoden für die Isolierung der Verbindungen der allgemeinen Formel (I) aus dem Reaktionsgemisch sowie für deren weitere Reinigung sind dem Fachmann bekannt. Anschliessend können die erhaltene Verbindung weiter verarbeitet werden.

Für das beschriebene Verfahren mit den Schritten (A)-(C) können als Lösungsmittel zahlreiche organische wasserfreie Verbindungen verwendet werden, wie beispielsweise Toluol, Benzin, Hexan, Heptan, tert.-Butylalkohol, Diethylether, Aceton, Benzol, Dioxan, Tetrahydrofuran, Chloroform, Dimethylformamid oder Pyridin.

Die folgenden Beispiele erläutern die Erfindung.

### Beispiel 1 (Herstellung eines α,β-ungesättigten Butyramids, d.i. But-2-enoic acid dimethylamide)

### Schritt 1A (Herstellung von Butyramid-Silylenolether, d.i. Dimethyl-(1-trimethylsilanyloxy-but-1-enyl)-amine)

Zu einer Lösung aus 10 g (0.085 Mol) N,N-Dimethylbutyramid und 54 g absolutem Tetrahydrofuran (THF) werden bei einer Innentemperatur von -80°C 46 ml einer 2 molaren (2M) Lithium-diisopropylamid-Lösung (LDA-Lösung) vorsichtig zu dosiert und etwa 1 Stunde bei -70 bis -80°C nachgerührt. Danach werden bei gleicher Innentemperatur 28 g (0:255 Mol) Trimethylchlorsilan zudosiert. Dabei scheidet sich LiCl ab. Nach Zugabe des Silans wird das Kältebad entfernt. Den Ansatz lässt man unter Stickstoff (N₂) über Nacht auf Umgebungstemperatur erwärmen. Bei einer Innentemperatur von 70-90°C wird das Reaktionsgemisch unter N₂-Fluss destilliert, dabei reichern sich ca. 8 g des gewünschten Silylenolethers in der Vorlage an.
¹H-NMR (200MHz, CDCl₃, δ): 3.48-3.38 (1H, t); 2.28(6H, s); 1.89-1.72(2H, m); 0.77 (3H, t); 0.02 (9H, s)

### Schritt 1B (Herstellung von α,β-ungesättigtemn Butyramid, d.i. But-2-enoic acid dimethylamide)

2 g (8 mMol) des Silylenolethers aus Stufe 1 werden unter Stickstoff mit 16 g absolutem Acetonitril, 2 g Chloroform, 2.9 g (0.024 Mol) Allylmethylcarbonat und 0.16 g (0.16 mMol) des Pd-Katalysators auf Rückflusstemperatur (Innentemperatur 75-80°C) erwärmt. Bereits beim Erwärmen setzt eine deutlich erkennbare Gasentwicklung ein. Die dunkelgrüne Lösung wird über Nacht gerührt. Die erhaltene schwarze Suspension wird filtriert und unter vermindertem Druck (nur bis p=80 mbar) eingeengt. Man erhält ca. 0.9 g ungesättigtes Butyramid.
¹H-NMR (200MHz, CDCl₃, δ) : 6.88-6.72 (1H, m); 6.20(1H, d breit); 3.04 (3H, s); 2.98 (3H, s); 1.78(3H, d);
MS (+EI): 114 (M+1, 40%); 98 (100%)

In analoger Art und Weise, wie eben beschrieben, lässt sich 4-Dimethylcarbamoyl-2,2-dimethyl-butyric acid methyl ester in 4-Dimethylcarbamoyl-2,2-dimethyl-but-3-enoic acid methyl ester umwandeln.

### Beispiel 2 (Herstellung von α,β-ungesättigten Valerolactam, d.i. 6-Oxo-3,6-dihydro-2H-pyridine-1-carboxylic acid tert-butyl ester)

### Schritt 2A (Herstellung von N-bocyliertem Valerolactam, d.i. 2-Oxo-piperidine-1-carboxylic acid tert-butyl ester)

Zu einer Lösung aus 10 g (0.097 Mol) δ-Valerolactam und 44.5 g absolutem THF werden bei einer Innentemperatur von -60°C 55 ml einer 2M LDA-Lösung vorsichtig zudosiert und etwa 1 Stunde bei -60 bis -70°C nachgerührt. Danach tropft man bei gleicher Innentemperatur eine Lösung bestehend aus 22.22 g (0.102 mol) Boc-Anhydrid und 18 g absolutem THF zu und lässt das Reaktionsgemisch über Nacht auf Umgebungstemperatur aufwärmen. Das erhaltene Gemisch wird zu einer Mischung bestehend aus 50 g Toluol und 100 g Wasser gegeben und ca. 30 Minuten gerührt. Die rote, organische Phase wird dreimal mit je 50 g Wasser gewaschen und dann destillativ soweit möglich unter vermindertem Druck eingeengt. Es resultieren 19 g eines dunklen Öls.
¹H-NMR (200MHz, CDCl₃, δ) : 3.78-3.55 (2H, t); 2.55-2.42(2H, t); 1.90-1.72 (4H, m); 1.57-1.48 (9H, s)
MS: 199 (M, <1%); 144 (46%); 57 (100%)

### Schritt 2B (Herstellung von Boc-Valerolactam-Silylenolether, d.i 6-Trimethylsilanyloxy-3,4-dihydro-2H-pyridine-1-carboxylic acid tert-butyl ester)

Zu einer Lösung bestehend aus 12 g (0.052 mol) N-Boc-Valerolactam aus Stufe 1 und 44.5 g absolutem THF werden bei einer Innentemperatur von -60°C 30 ml einer 2M LDA-Lösung vorsichtig zudosiert und etwa 1 Stunde bei -60°C bis -70°C nachgerührt. Danach werden bei gleicher Innentemperatur 6.2 g (0.057 mol) Trimethylchlorsilan zugegeben. Dabei scheidet sich LiCl ab. Nach Zugabe des Silans wird das Kältebad entfernt. Den Ansatz lässt man unter N₂ über Nacht auf Umgebungstemperatur erwärmen. Die Reaktionsmischung wird dann in ein Gemisch bestehend aus 50 g Toluol und 50 g Wasser gegossen, kurz gerührt und die organische Phase dreimal mit je 50 g Wasser gewaschen. Nach dem Einengen bleiben 14 g eines klaren Öls im Kolben zurück.
¹H-NMR (200MHz, CDCl₃, δ): 4.12 (1H, t) ; 3.03 (2H, t) ; 1.92-1.81 (2H, m); 1.55-1.40 (2H, m); 1.27(9H, s); 0.01 (9H, s)

### Schritt 2C (α,β-ungesättigtes Valerolactam, d.i. 6-Oxo-3,6-dihydro-2H-pyridine-1-carboxylic acid tert-butyl ester)

2g (0.0074 Mol) des Silylenolethers aus Stufe 2 werden zusammen mit 25 g absolutem Acetonitril, 0.4 g Pd-Katalysator und 0.8 g p-Benzochinon über Nacht bei Raumtemperatur gerührt. Die erhaltene schwarze Reaktionsmischung wird mit 50 g 5%iger NaOH-Lösung intensiv gerührt, mit 50g Toluol extrahiert und die organische Phase soweit möglich eingeengt. Es bleibt 1 g eines leicht beweglichen, dunklen Öls im Kolben zurück.
¹H-NMR (200MHz, CDCl₃, δ): 6.82-6.72 (1H, m) ; 5.97(1H, d); 3.88 (2H, t); 2.46-2.35 (2H, m); 1.54 (9H, s)

### Beispiel 3 (N,N-Diethyl-3-phenyl-acrylamide)

### Schritt 3A (N,N-Diethyl-(3-phenyl-1-trimethylsilanyloxypropenyl)-amine)

In 15 ml Tetrahydrofuran werden unter Kühlung (-78°C) 3.3 ml Diisopropylamin vorgelegt und mit 9.25 mL 2.5 M Hexyllithium Lösung versetzt. Nach 30 min werden 4.11 g (20 mmol) N,N-Diethyl-3-phenyl-propionamid zugegeben. Nach weiteren 60 Minuten wurden 7.6 mL Trimethylchlorsilan zugegeben. Über Nacht wird die Reaktionsmischung auf Raumtemperatur erwärmen gelassen. Unter vermindertem Druck (ca. 0.6 mbar) werden destillativ bei 125-128°C 3.55 g (64% der Theorie) N,N- Diethyl-(3-phenyl-1-trimethylsilanyloxy-propenyl)-amine (Zwischenprodukt) erhalten, welches ohne weitere Reinigung im Schritt 2 eingesetzt werden kann.

### Schritt 3B (N,N-Diethyl-3-phenyl-acrylamide)

Eine Lösung aus 20 mL Acetonitril, 1.35 mL Chloroform, 2.63 ml Allylmethylcarbonat, 0.15 g Tris-(dibenzylidenaceton)-dipalladium Chloroform Komplex und 2.1 g des eben hergestellten Zwischenproduktes werden über Nacht am Rückfluss gekocht. Die Reaktionsmischung wird klarfiltriert und im Vakuum eingeengt. Der verbleibende Rückstand enthält 1.2 g N,N-Diethyl-3-phenyl-acrylamide.
MS (eI) : 204 (5%), 203 (M⁺, 35%), 188 (18%), 131 (100%)

### Beispiel 4 (Azacyclotridec-3-en-2-on)

### Schritt 4A (4,5,6,7,8,9,10,11,12,13-Decahydro-1-oxa-3a-aza-cyclopentacyclotridecene-2,3-dione)

Bei Raumtemperatur wird zu einer Lösung von 19.7 g (0.1 mol) Laurinolactam in 400 mL Toluol 19 g (0.13 mol) Oxalylchlorid gegeben. Das Reaktionsgemisch wird 3 Stunden bei 55°C gerührt und anschliessend im Vakuum eingeengt. Der Rückstand wird mit 300 mL Heptan kristallisiert. Es werden 21.4 g (85% d. Th.) Zwischenprodukt (4,5,6,7,8,9,10,11,12,13-Decahydro-1-oxa-3a-azacyclopentacyclotridecene-2,3-dione) erhalten.
¹H-NMR (200MHz, CDCl₃, δ): 5.11 (2H, t); 3.73-3.67 (2H, m); 2.25-2.20 (2H, m); 1.70-1.29 (16H, m).
¹³C-NMR (50MHz, CDCl₃, δ): 156.4; 151.6; 142.0; 40.4; 28.4; 27.4; 26.8; 26.7; 25.9; 25.4; 24.4; 24.1; 23.9.

### Schritt 4B (Azacyclotridec-3-en-2-on)

Ein Gemisch aus 1.0 g(4 mmol) des eben hergestellten Zwischenproduktes (4,5,6,7,8,9,10,11,12,13-Decahydro-1-oxa-3a-aza-cyclopentacyclotridecene-2,3-dione), 1.4 g Allylmethylcarbonat, 5.9 g Chloroform, 0.1 g Tris-(dibenzylidenaceton)-dipalladium Chloroform Komplex und 10 mL Acetonitril wird am Rückfluss gekocht. Nach 4 Stunden und 16 Stunden werden weitere 0.1 g Tris-(dibenzylidenaceton)-dipalladium Chloroform Komplex zugegeben. Die Reaktionslösung wird nach 2 Tagen kochen eingeengt, in 20 mL Methanol gelöst und bei 0°C mit 8 mmol Natriummethylat (gelöst in 1.5 mL Methanol) während 1 Stunde verrührt, dann im Vakuum eingeengt. Der Rückstand wird mit Essigsäureethylester verdünnt und mit 1 N Salzsäure gewaschen. Die organische Phase wird eingeengt. Es verbleiben 1,2 g Rückstand bestehend aus 6% Laurinolactam, 5% Azacyclotridec-3-en-2-on, 11% 3-Allyl-azacyclotridecan-2-on, 47% Zwischenprodukt (4,5,6,7,8,9,10,11,12,13-Decahydro-1-oxa-3a-aza-cyclopentacyclotridecene-2,3-dione), 25% 19-Allyl-4,5,6,7,8,9,-10,11,12,13-decahydro-1-oxa-3a-aza-cyclopentacyclotridecene-2,3-dione und 6% Dibenzylidenaceton. Die Produkte werden mittels Chromatographie (Kieselgel, Essigsäureethylester/Hexan) getrennt.
Azacyclotridec-3-en-2-on: MS (eI) : 195 (M⁺, 18%), 167 (18%), 152 (18%), 150 (20%), 124 (46%), 81 (100%). 3-Allyl-azacyclotridecan-2-on: MS (eI): 237 (M⁺, 50%), 207 (38%), 196 (65%), 55 (100%).
14-Allyl-4,5,6,7,8,9,10,11,12,13-decahydro-1-oxa-3a-aza-cyclopentacyclotridecene-2,3-dione: ¹H-NMR (200MHz, CDCl₃, δ): 5.5.3 (1H, ddt), 5.09 (1H, d); 5.08 (1H, d); 3.85 (1H, ddd); 3.76 (1H, ddd); 2.57 (1H, dd); 2.49 (1H, dd); 2.56-1.00 (18H, m).
MS (eI) : 250 (M⁺-allyl, 80%), 207 (100%).

In analoger Art und Weise lässt sich aus Octahydro-quinolin-2-on die entsprechende α,β-ungesättigte Verbindung 4a,5,6,7,8,8a-Hexahydro-1H-quinolin-2-on erhalten.

## Patentansprüche

1. Verfahren zur Herstellung von α,β-ungesättigten Amidverbindungen der allgemeinen Formel (I): 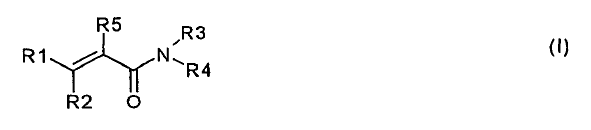 worin
R₁ und R₂ unabhängig voneinander Wasserstoff; gegebenenfalls mit Hydroxy, Halogen, Phenyl, substituiertem Phenyl, oder mit einer Estergruppe [-C(O)OAlkyl] oder einer Amidgruppe [-C(O)NH₂ oder -C(O)NHAlkyl], substituiertes lineares oder verzweigtes (C₁-C₁₈)-Alkyl oder (C₁-C₁₈)-Alkenyl; gegebenenfalls mit Halogen substituiertes Phenyl; oder
entweder R₁ oder R₂ einen Rest Y-R₆; worin
Y Sauerstoff (-O-); Schwefel (-S-); -NR₇-; oder Dialkylsilyloxy [-(Alkyl)₂Si-O-];
R₆ Wasserstoff, gegebenenfalls mit Hydroxy, Halogen, Phenyl, substituiertem Phenyl oder mit einer Estergruppe [-C(O)OAlkyl] oder einer Amidgruppe [-C(O)NH₂] oder [-C(O)NHAlkyl], substituiertes lineares oder verzweigtes (C₁-C₁₈)-Alkyl; gegebenenfalls mit Halogen substituiertes Phenyl;
R₇ (C₁-C₁₈)-Alkyl oder -N(R₆) (R₇) einen 5- oder 6-gliedrigen heterocyclischen Ring; oder
R₁ zusammen mit R₃ eine direkte Bindung oder einen Rest der Formel -(CH₂)ₙ-; worin
n eine ganze Zahl von 1 bis 12; oder
R₁ zusammen mit R₂ Cyclohexyliden; oder
R₁ zusammen mit R₅ und der eingeschlossenen (C=C)-Doppelbindung Cyclohexenyl; oder
R₁ zusammen mit R₅ und der eingeschlossenen (C=C)-Doppelbindung einen Rest eines einfach-ungesättigten Bicyclischen Ringes;
R₃ Wasserstoff, gegebenenfalls durch Phenyl, Hydroxyl, oder Halogen substituiertes lineares oder verzweigtes (C₁-C₁₂)-Alkyl, welches gegebenenfalls durch ein oder mehrere Sauerstoffatome unterbrochen ist, (C₅-C₈)-Cycloalkyl oder (C₅-C₈)-Cycloalkenyl, welche gegebenenfalls durch ein oder mehrere Sauerstoffatome unterbrochen sind; gegebenenfalls durch Halogen oder Hydroxyl substituiertes Phenyl; oder R₃ zusammen mit R₁ eine direkte Bindung oder einen Rest der Formel -(CH₂)ₙ-;
R₄ eine der Bedeutungen von R₃, vorzugsweise Wasserstoff, gegebenenfalls durch Phenyl, Hydroxyl, oder Halogen substituiertes lineares oder verzweigtes (C₁-C₁₂)-Alkyl, gegebenenfalls durch Halogen oder Hydroxyl substituiertes Phenyl; oder -NR₃R₄ einen 5- oder 6-gliedrigen heterocyclischen Ring; bedeuten und
R₅ eine der für R₁ oder R₂ als unabhängige Substituenten angegebenen Bedeutungen hat,
welches **dadurch gekennzeichnet ist, dass** man
(A) in einer Verbindung der allgemeinen Formel (II): 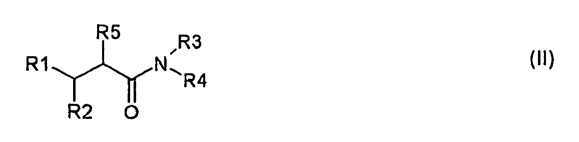 worin R₁, R₂, R₃, R₄ und R₅ entsprechend die oben angegebenen Bedeutungen haben und [die Verbindung der Formel (II)] die entsprechende gesättigte Verbindung darstellt, Schutzgruppen einführt, so dass eine Verbindung der allgemeinen Formel (III) entsteht: 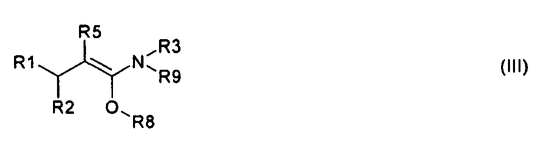 worin
R₈ Trialkylsilyl, oder (wenn R₄ = Wasserstoff) zusammen mit R₉ den Rest -C(O)-(CH₂)ₘ-C(O)- und
R₉ (wenn R₄ = Wasserstoff) Alkyloxycarbonyl oder Phenyloxycarbonyl, vorzugsweise Boc (= tert.-Butyloxycarbonyl); oder Trialkylsilyl, oder zusammen mit R₈ den Rest -C(O)-(CH₂)ₘ-C(O)-, und
m 0, 1, 2, oder 3, vorzugsweise 0 oder 1, vorzugsweise 0, bedeuten,
und worin für den Fall, dass in der Verbindung der allgemeinen Formel (II) Hydroxyl anwesend ist, dieses gegebenenfalls mit einer monovalenten Schutzgruppe R₈ und/oder R₉ reagiert hat;
(B) die [gemäss Schritt (A)] erhaltene Verbindung in Gegenwart (i) eines Dehydrierungskatalysators ausgewählt aus Verbindungen der Gruppe der Übergangsmetalle des Periodensystems der Elemente, vorzugsweise aus Verbindungen der Metalle der VIII. Gruppe des Periodensystems, und in Gegenwart (ii) eines Oxidationsmittels ausgewählt aus der Gruppe enthaltend gegebenenfalls substituiertes Benzochinon, Allylmethylcarbonat, Allylethylcarbonat und/oder Allylpropylcarbonat, umsetzt, wobei die α,β-Doppelbindung in die α,β-Stellung eingeführt wird, und
(C) gegebenenfalls anwesende Schutzgruppen R₈, sowie den Substituent R₉ entfernt.

2. Verfahren nach Anspruch 1, **dadurch gekennzeichnet, dass** R₁ und R₂ unabhängig voneinander Wasserstoff, gegebenenfalls mit Hydroxy, Phenyl, mit Halogen oder Hydroxy substituiertem Phenyl, oder mit einer (C₁₋₄)Alkyl-Estergruppe oder einer Amidgruppe oder (C₁₋₄)Alkyl-Amidgruppe substituiertes lineares oder verzweigtes (C₁-C₈)-Alkyl oder (C₁-C₈)-Alkenyl, gegebenenfalls mit Halogen substituiertes Phenyl; vorzugsweise lineares oder verzweigtes (C₁-C₈) -Alkyl oder (C₁-C₈)-Alkenyl, Benzyl oder Phenyl, bedeuten.

3. Verfahren nach Anspruch 1, **dadurch gekennzeichnet, dass** R₂ Wasserstoff und R₁ lineares oder verzweigtes (C₁-C₈)-Alkyl oder (C₁-C₈)-Alkenyl; Benzyl oder Phenyl oder Y-R₆ bedeuten.

4. Verfahren nach Anspruch 1, **dadurch gekennzeichnet, dass** R₁ Wasserstoff und R₂ lineares oder verzweigtes (C₁-C₈)-Alkyl oder (C₁-C₈)-Alkenyl; Benzyl oder Phenyl oder Y-R₆ bedeuten.

5. Verfahren nach Anspruch 1, **dadurch gekennzeichnet, dass** R₁ zusammen mit R₃ die direkte Bindung oder einen Rest der Formel -(CH₂)ₙ- und n eine ganze Zahl von 1 bis 12; oder R₁ zusammen mit R₂ Cyclohexyliden; oder R₁ zusammen mit R₅ Cyclohexenyl, bedeuten.

6. Verfahren nach Anspruch 1, **dadurch gekennzeichnet, dass** Y (im Rest Y-R₆) Sauerstoff bedeutet.

7. Verfahren nach Anspruch 1, **dadurch gekennzeichnet, dass** R₆ Wasserstoff, gegebenenfalls mit Hydroxy, Halogen, Phenyl, mit Halogen substituiertem Phenyl, oder mit einer (C₁₋₄)Alkyl-Estergruppe oder einer Amidgruppe oder einer (C₁₋₄)Alkyl-Amidgruppe substituiertes lineares oder verzweigtes (C₁-C₈)-Alkyl; gegebenenfalls mit Halogen substituiertes Phenyl; vorzugsweise Wasserstoff, gegebenenfalls mit Phenyl, oder mit einer (C₁₋₄)Alkyl-Estergruppe oder einer Amidgruppe oder einer (C₁₋₄)Alkyl-Amidgruppe substituiertes lineares oder verzweigtes (C₁-C₈)-Alkyl; oder Phenyl; vorzugsweise Wasserstoff, lineares oder verzweigtes (C₁-C₈)-Alkyl oder Phenyl, bedeutet.

8. Verfahren nach Anspruch 1, **dadurch gekennzeichnet, dass** der Substituent -N(R₆) (R₇) als heterocyclischen Ring einen Rest von Pyrrolidin oder Piperidin bedeutet.

9. Verfahren nach Anspruch 1, **dadurch gekennzeichnet, dass** die Verbindung der Formel (II) ein Lactam einer omega-Amino-Fettsäure darstellt, vorzugsweise der omega-Aminobuttersäure, der omega-Aminovaleriansäure, der omega-Aminocapronsäure, oder der omega-Aminolaurinsäure.

10. Verfahren nach Anspruch 1, **dadurch gekennzeichnet, dass** in der Verbindung der Formel (I) R₁ zusammen mit R₅ und der eingeschlossenen (C=C)-Doppelbindung einen Rest eines einfach ungesättigten bicyclischen Ringes bedeuten, vorzugsweise ein gegebenenfalls durch Hydroxyl oder Amino substituierter Norbornylrest, vorzugsweise ein Norbornylrest.

11. Verfahren nach eine der Ansprüche 1-10, **dadurch gekennzeichnet, dass** R₃ und R₄ unabhängig voneinander Wasserstoff, gegebenenfalls durch Phenyl substituiertes lineares oder verzweigtes (C₁-C₄)-Alkyl, Phenyl; oder der Rest -NR₃R₄ einen Rest von Pyrrolidin oder Piperidin bedeuten.

12. Verfahren nach Anspruch 1, **dadurch gekennzeichnet, dass** R₅ Wasserstoff, tert.-Butyl oder gegebenenfalls durch Halogen oder Hydroxyl substituiertes Phenyl, vorzugsweise Wasserstoff; und R₈ Trimethylsilyl oder R₈ zusammen mit R₉ den Rest -C(O)-(CH₂)ₘ-C(O)-; oder R₉ Boc, Trimethylsilyl, oder R₉ zusammen mit R₈ den Rest -C(O)-(CH₂)ₘ-C(O)-, worin m 0, 1, 2, oder 3, vorzugsweise 0 oder 1, vorzugsweise 0, bedeutet.

13. Verfahren nach Anspruch 1, **dadurch gekennzeichnet, dass** R₉ als Alkyloxycarbonyl Isobutyloxycarbonyl, tert.-Butyloxycarbonyl, tert.-Amyloxycarbonyl, Cyclobutyloxycarbonyl, 1-Methylcylobutyloxycarbonyl, Cyclopentyloxycarbonyl, Cyclohexyloxycarbonyl, 1-Methylcyclohexyl, vorzugsweise tert.-Butyloxycarbonyl, bedeutet.

14. Verfahren nach einem der Ansprüche 1-13, **dadurch gekennzeichnet, dass** der Dehydrierungskatalysator [in Schritt (B)] ausgewählt ist aus Verbindungen (Salze und Komplexe) von Eisen, Ruthenium und Osmium; Cobalt, Rhodium, und Iridium; Nickel, Palladium und Platin; Kupfer, Silber und Gold vorzugsweise von Verbindungen auf der Basis von Rhodium, Palladium und Platin.

15. Verfahren nach Anspruch 14, **dadurch gekennzeichnet, dass** der Dehydrierungskatalysator eine Palladiumverbindung darstellt, vorzugsweise eine Pd(0)-Verbindung, vorzugsweise einen Tris(dibenzylidenaceton)diPalladium-ChloroformKomplex oder eine Pd(II)-Verbindung, vorzugsweise PdC1₂, Pd(dppe)₂, Pd(dppe)Cl₂, Pd(OAc)₂, Pd(dppe)(OAc)₂, n-Allyl-Pd-Komplexe, vorzugsweise π-Allyl-Pd-chlorid Dimer.

16. Verfahren nach einem der Ansprüche 1-15, **dadurch gekennzeichnet, dass** zur termischen Stabilisierung des Palladium-Komplexes ein zusätzlicher Komplexbildner zugesetzt wird, vorzugsweise 2,2'-Bipyridyl oder 1,10-Phenanthrolin.

17. Verfahren nach einem der Ansprüche 1-16, **dadurch gekennzeichnet, dass** als Chinon ein substituiertes Chinon verwendet wird, vorzugsweise ein durch C₁₋₄-Alkyl, Halogen, Cyano oder Nitro substituiertes Chinon.

## Claims

1. Method for the production of α,β-unsaturated amide compounds having the general formula (I): 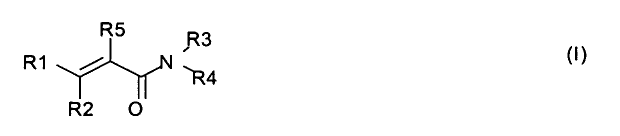 wherein,
R₁ and R₂ are independently hydrogen; optionally linear or branched (C₁-C₁₈) alkyl or (C₁-C₁₈) alkenyl substituted with hydroxy, halogen, phenyl, substituted phenyl, or an ester group [-C(O)Oalkyl] or an amide group [-C(O)NH₂ or - C(O)NHalkyl]; optionally phenyl substituted with halogen; or
R₁ or R₂ comprises a group Y-R₆; in which
Y is oxygen (-O-); sulphur (-S-); -NR₇-; or dialkylsilyloxy [-(alkyl)₂Si-O-];
R₆ is hydrogen, optionally linear or branched (C₁-C₁₈) alkyl substituted with hydroxy, halogen, phenyl, substituted phenyl or with an ester group [-C(O)OAlkyl] or an amide group [-C(O)NH₂] or [-C(O)NHAlkyl]; optionally phenyl substituted with halogen;
R₇ is (C₁-C₁₈) alkyl or -N(R₆)(R₇) is a 5- or 6-membered heterocyclic ring;
or
R₁ together with R₃ is directly bonded or a group having the formula -(CH₂)ₙ-; in which
n is a whole number from 1 to 12;
or
R₁ together with R₂ is cyclohexylidene;
or
R₁ together with R₅ and the incorporated (C=C)-double bond is cyclohexenyl;
or
R₁ together with R₅ and the incorporated (C=C)-double bond forms a group of a monounsaturated bicyclic ring;
R₃ is hydrogen, optionally a linear or branched (C₁-C₁₂) alkyl substituted with phenyl, hydroxyl, or halogen, carrying one or more oxygen atoms, (C₅-C₈)-cycloalkyl or (C₅-C₈)-cycloalkenyl, carrying one or more oxygen atoms; preferably, phenyl substituted with halogen or hydroxyl; or R₃ together with R₁ is directly bond or forms a group of the formula -(CH₂)ₙ-;
R₄ has one of the meanings of R₃, preferably hydrogen, optionally linear or branched (C₁-C₁₂) alkyl substituted with phenyl, hydroxyl, or halogen, optionally phenyl substituted with halogen or hydroxyl; or -NR₃R₄ a 5- or 6-membered heterocyclic ring; and
R₅ has one of the meanings specified for R₁ or R₂ as independent substituents,
wherein said method comprises the steps of:
(A) reacting a compound of the general formula (II): 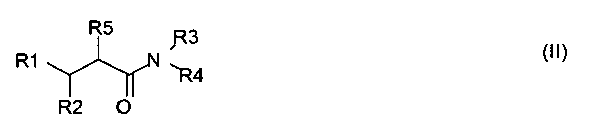 wherein R₁, R₂, R₃, R₄ and R₅ have the meanings specified above and [the compound of formula (II)]represent the corresponding saturated compound, to introduce protective groups so as to produce a compound of the general formula (III): 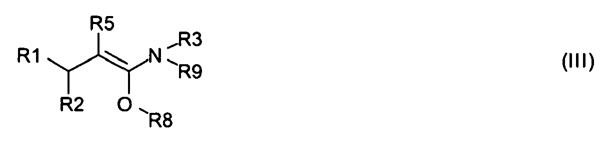 wherein R₈ is trialkylsilyl, or (when R₄ = hydrogen) together with R₉ forms the group -C(O)-(CH₂)ₘ-C(O)- and
R₉ (when R₄ = hydrogen) is alkyloxycarbonyl or phenyloxycarbonyl, preferably Boc (= tert. butyloxycarbonyl); or trialkylsilyl, or together with R₈ the group -C(O)-(CH₂)ₘ-C(O)-, and
m is 0, 1, 2, or 3, preferably 0 or 1, preferably 0,
and in the case in which for the compound of the general formula (II) hydroxyl is present, it is reacted, with a monovalent protective group R₈ and/or R₉;
(B) reacting the compound obtained in step (A) in presence of (i) a dehydrogenation catalyst selected from compounds of the metal-transition group of the periodic element system, preferably from metal compounds of the Group VIII of the periodic system, and in presence of (ii) an oxidising agent selected from the group comprising optionally substituted benzoquinone, allyl methyl carbonate, allyl ethyl carbonate and/or allyl propyl carbonate,
to introduce an α,β-double bond in the α,β-position, and
(C) optionally removing, if present, the protective groups R₈, as well as the substituent R₉.

2. Method according to claim 1, wherein R₁ and R₂ are independently hydrogen, optionally linear or branched (C₁-C₈) alkyl or (C₁-C₈) alkenyl substituted with hydroxy, phenyl, phenyl substituted with halogen or hydroxy, or with a (C₁₋₄) alkyl ester group or an amide group or (C₁₋₄) alkyl amide group, preferably, phenyl substituted with halogen; preferably linear or branched (C₁-C₈) alkyl or (C₁-C₈) alkenyl, benzyl or phenyl.

3. Method according to claim 1, wherein R₂ is hydrogen and R₁ is linear or branched (C₁-C₈) alkyl or (C₁-C₈) alkenyl, benzyl or phenyl or Y-R₆.

4. Method according to claim 1, wherein R₁ is hydrogen and R₂ is linear or branched (C₁-C₈) alkyl or (C₁-C₈) alkenyl; benzyl or phenyl or Y-R₆.

5. Method according to claim 1, wherein R₁ together with R₃ is directly bonded or forms a group of the formula-(CH₂)ₙ- and n is a whole number from 1 to 12; or R₁ together with R₂ is cyclohexylidene; or R₁ together with R₅ is cyclohexenyl.

6. Method according to claim 1, wherein Y in the group Y-R₆ is oxygen.

7. Method according to claim 1 wherein R₆ is hydrogen, optionally linear or branched (C₁-C₈) alkyl or phenyl substituted with hydroxy, halogen, phenyl, phenyl substituted with halogen, or an (C₁₋₄)alkyl ester group or an amide group or a (C₁₋₄)alkyl amide group; optionally phenyl substituted with halogen; preferably hydrogen, optionally linear or branched (C₁-C₈) alkyl substituted with phenyl, or with a (C₁₋₄) alkyl ester group or an amide group or a (C₁₋₄) alkyl amide group; or phenyl; preferably hydrogen, linear or branched (C₁-C₈) alkyl or phenyl.

8. Method according to claim 1, wherein the substituent -N(R₆)(R₇) as heterocyclic ring is a pyrrolidine or piperidine.

9. Method according to claim 1, wherein the compound of the formula (II) represents a lactam of an omega amino fatty acid, preferably aminobutyric acid, omega aminovaleric acid, omega aminocapronic acid, or omega aminolauric acid.

10. Method according to claim 1, wherein the compound of the formula (I), R₁ together with R₅ and the incorporated (C=C)-double bond represent a monounsaturated bicyclic ring, preferably a norbornyl group optionally substituted with hydroxyl or amino, preferably a norbornyl group.

11. Method according to any of claims 1 to 10 wherein R₃ and R₄ are independently hydrogen, linear or branched (C₁-C₄) alkyl optionally substituted with phenyl, phenyl; or the group -NR₃R₄ is pyrrolidine or piperidine.

12. Method according to claim 1, wherein R₅ is hydrogen, tert. butyl or optionally phenyl substituted with halogen or hydroxyl, preferably hydrogen; and R₈ is trimethylsilyl or R₈ together with R₉ is the group -C(O)-(CH₂)ₘ-C(O)-; or R₉ is Boc, trimethylsilyl, or R₉ together with R₈ is the group -C(O)-(CH₂)ₘ-C(O)-, in which m is 0, 1, 2, or 3, preferably 0 or 1, preferably 0.

13. Method according to claim 1, wherein R₉ is alkyloxycarbonyl, isobutyloxycarbonyl, tert. butyloxycarbonyl, tertiary amyloxycarbonyl, cyclobutyloxycarbonyl, 1-methylcylobutyloxycarbonyl, cyclopentyloxycarbonyl, cyclohexyloxycarbonyl, 1-methylcyclohexyl, preferably tertiary butyloxycarbonyl.

14. Method according to one of the claims 1-13, wherein the dehydrogenation catalyst [in step (B)] is selected from amongst compounds (salts and complexes) iron, ruthenium and osmium; cobalt, rhodium, and iridium; nickel, palladium and platinum; copper, silver and gold preferably from compounds based on rhodium, palladium and platinum.

15. Method according to claim 14, wherein the dehydrogenation catalyst is a palladium compound, preferably a Pd(0) compound, preferably a tris(dibenzylidene acetone) dipalladium chloroform complex or a Pd(II) compound, preferably PdCl₂, Pd(dppe)₂, Pd(dppe)Cl₂, Pd(OAc)₂, Pd(dppe)(OAc)₂, x-allyl Pd complex, preferably π-allyl Pd chloride dimer.

16. Method according to one of the claims 1-15, wherein an additional complexing agent is used for the thermal stabilisation of the palladium complex, preferably 2,2'-bipyridyl or 1,10-phenanthroline.

17. Method according to one of the claims 1-16; wherein the quinone is a substituted quinone, preferably a quinone substituted with C₁₋₄ alkyl, halogen, cyano or nitro.

## Revendications

1. Procédé de production de composés amide α, β-insaturés de la formule générale (1): 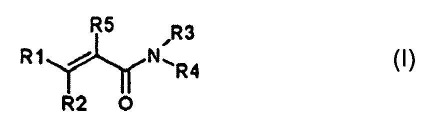 dans laquelle
R₁ et R₂ représentent indépendamment l'un de l'autre hydrogène ; optionnellement phényle substitué par hydroxy, halogène, phényle ou alkyle (C₁-C₁₈) ou alcényle (C₁-C₁₈) linéaire ou ramifié substitué par un groupe ester [-C(O)Oalkyle] ou un groupe amide [-C(O)NH₂ ou -C(O)NHalkyle] ; optionnellement phényle substitué par halogène ;
ou
soit R₁ soit R₂ représentent un reste Y-R₆ ; où
Y représente oxygène (-O-) ; soufre (-S-) ; -NR₇-; ou dialkylsilyloxy [-(alkyle)₂Si-O-] ;
R₆ représente hydrogène, optionnellement phényle substitué par hydroxy, halogène, phényle ou alkyle (C₁-C₁₈) linéaire ou ramifié substitué par un groupe ester [-C(O)Oalkyle] ou un groupe amide [-C(O)NH₂] ou [-C(O)NHalkyle] ; optionnellement phényle substitué par halogène ;
R₇ représente alkyle (C₁-C₁₈) ou -N(R₆)(R₇) un anneau hétérocyclique à 5 ou 6 éléments ;
ou
R₁ ensemble avec R₃ représentent une liaison directe ou un reste de la formule -(CH₂)ₙ- ; dans laquelle
n est un nombre entier de 1 à 12 ;
ou
R₁ ensemble avec R₂ représentent cyclohexylidène ;
ou
R₁ ensemble avec R₅ et la double liaison (C=C) enfermée représentent cyclohexényle ;
ou
R₁ ensemble avec R₅ et la double liaison (C=C) enfermée représentent un reste d'un anneau bicyclique mono-insaturé ;
R₃ représente hydrogène, optionnellement alkyle (C₁-C₁₂) linéaire ou ramifié substitué par phényle, hydroxyle ou halogène, qui est optionnellement interrompu par un ou plusieurs atomes d'oxygène, cycloalkyle (C₅-C₈) ou cycloalcényle (C₅-C₈) qui sont optionnellement interrompus par un ou plusieurs atomes d'oxygène ; optionnellement phényle substitué par halogène ou hydroxyle ; ou R₃ ensemble avec R₁ représentent une liaison directe ou un reste de la formule -(CH₂)ₙ- ;
R₄ représente une des significations de R₃, de préférence hydrogène, optionnellement alkyle (C₁-C₁₂) linéaire ou ramifié substitué par phényle, hydroxyle ou halogène, optionnellement phényle substitué par halogène ou hydroxyle ; ou
-NR₃R₄ représente un anneau hétérocyclique à 5 ou 6 éléments et
R₅ a une des significations indiquées pour R₁ ou R₂ en tant que substituants indépendants,
**caractérisé en ce que** (A) dans un composé de la formule générale (II) : 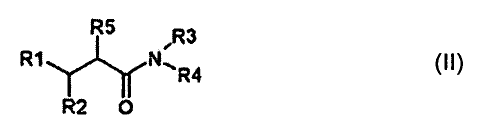 dans laquelle R₁, R₂, R₃, R₄ et R₅ ont les significations indiquées ci-dessus et [le composé de la formule (II)] représente le composé saturé correspondant, sont introduits des groupes protecteurs de manière à obtenir un composé de la formule générale (III): 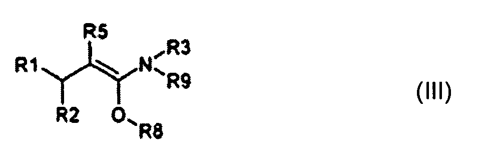 dans laquelle
R₈ représente trialkylsilyle, ou (si R₄ = hydrogène) ensemble avec R₉ le reste -C(O)-(CH₂)ₘ-C(O)- et
R₉ représente (si R₄ = hydrogène) alkyloxycarbonyle ou phényloxycarbonyle, de préférence boc (= tert.-butyloxycarbonyl) ; ou trialkylsilyle, ou ensemble avec R₈ le reste -C(O)-(CH₂)ₘ-C(O)-, et
m est égal à 0, 1, 2 ou 3, de préférence 0 ou 1, de préférence 0,
et dans laquelle au cas où dans le composé de la formule générale (II) est présent de l'hydroxyle, celui-ci a réagi optionnellement avec un groupe protecteur monovalent R₈ et/ou R₉ ;
(B) le composé obtenu [selon l'étape (A)] est réagi en présence (i) d'un catalyseur de déshydrogénation choisi parmi des composés du groupe des métaux de transition du tableau périodique des éléments, de préférence parmi des composés des métaux du groupe VIII du tableau périodique, et en présence (ii) d'un agent oxydant choisi dans le groupe comprenant optionnellement benzoquinone substituée, allylméthylcarbonate, allyléthylcarbonate et/ou allylpropylcarbonate, où la double liaison α, β est introduite dans la position α, β, et
(C) des groupes protecteurs R₈ optionnellement présents ainsi que le substituant R₉ sont enlevés.

2. Procédé selon la revendication 1, **caractérisé en ce que** R₁ et R₂ représentent indépendamment l'un de l'autre hydrogène ; optionnellement phényle substitué par hydroxy, phényle, par halogène ou hydroxy, ou un alkyle (C₁-C₈) ou alcényle (C₁-C₈) linéaire ou ramifié substitué par un groupe ester alkyle (C₁₋₄) ou un groupe amide ou un groupe amide alkyle (C₁₋₄) ; optionnellement phényle substitué par halogène ; de préférence alkyle (C₁-C₈) ou alcényle (C₁-C₈) linéaire ou ramifié, benzyle ou phényle.

3. Procédé selon la revendication 1, **caractérisé en ce que** R₂ représente hydrogène et R₁ représente alkyle (C₁-C₈) ou alcényle (C₁-C₈) linéaire ou ramifié ; benzyle ou phényle ou Y-R₆.

4. Procédé selon la revendication 1, **caractérisé en ce que** R₁ représente hydrogène et R₂ représente alkyle (C₁-C₈) ou alcényle (C₁-C₈) linéaire ou ramifié ; benzyle ou phényle ou Y-R₆.

5. Procédé selon la revendication 1, **caractérisé en ce que** R₁ ensemble avec R₃ représentent la liaison directe ou un reste de la formule - (CH₂)ₙ- et n est un nombre entier de 1 à 12 ; ou R₁ ensemble avec R₂ représentent cyclohexylidène ; ou R₁ ensemble avec R₅ représentent cyclohexényle.

6. Procédé selon la revendication 1, **caractérisé en ce que** Y (dans le reste Y-R₆) représente oxygène.

7. Procédé selon la revendication 1, **caractérisé en ce que** R₆ représente hydrogène, optionnellement phényle substitué par hydroxy, halogène, phényle, par halogène ou un alkyle (C₁-C₈) linéaire ou ramifié substitué par un groupe ester alkyle (C₁-C₄) ou un groupe amide ou un groupe amide alkyle (C₁-C₄); optionnellement phényle substitué par halogène ; de préférence hydrogène, optionnellement alkyle (C₁-C₈) linéaire ou ramifié substitué par phényle, ou par un groupe ester alkyle (C₁-C₄) ou un groupe amide ou un groupe amide alkyle (C₁-C₄) ; ou phényle ; de préférence hydrogène, alkyle (C₁-C₈) linéaire
ou ramifié ou phényle.

8. Procédé selon la revendication 1, **caractérisé en ce que** le substituant -N(R₆)(R₇) sous forme d'anneau hétérocyclique représente un reste de pyrrolidine ou pipéridine.

9. Procédé selon la revendication 1, **caractérisé en ce que** le composé de la formule (II) représente un lactame d'un acide gras aminé oméga, de préférence de l'acide aminobutyrique oméga, de l'acide aminovalérianique oméga, de l'acide aminocaproïque oméga ou de l'acide aminolaurique oméga.

10. Procédé selon la revendication 1, **caractérisé en ce que** dans le composé de la formule (I) R₁ ensemble avec R₅ et la double liaison (C=C) enfermée représentent un reste d'un anneau bicyclique mono-insaturé, de préférence un reste de norbornyle optionnellement substitué par hydroxyle ou amino, de préférence un reste de norbornyle.

11. Procédé selon l'une des revendications 1 à 10, **caractérisé en ce que** R₃ et R₄ représentent indépendamment l'un de l'autre hydrogène, optionnellement alkyle (C₁-C₄) linéaire ou ramifié substitué par phényle, phényle ; ou le reste -NR₃R₄ représente un reste de pyrrolidine ou de pipéridine.

12. Procédé selon la revendication 1, **caractérisé en ce que** R₅ représente hydrogène, butyle tertiaire ou optionnellement phényle substitué par halogène ou hydroxyle, de préférence hydrogène ; et R₈ représente triméthylsilyle et R₈ ensemble avec R₉ représentent le reste -(C(O)-(CH₂)ₘ-C(O)-; ou R₉ représente boc, triméthylsilyle, ou R₉ ensemble avec R₈ représentent le reste -(C(O)-(CH₂)ₘ-C(O)-, où m est égal à 0, 1, 2 ou 3, de préférence 0 ou 1, de préférence 0.

13. Procédé selon la revendication 1, **caractérisé en ce que** R₉ en tant qu'alkyloxycarbonyle représente isobutyloxycarbonyle, tert.-butyloxycarbonyle, tert.-amyloxycarbonyle, cyclobutyloxycarbonyle, 1-méthylcyclobutyloxycarbonyle, cyclopentyloxycarbonyle, cyclohéxyloxycarbonyle, 1-méthylcyclohéxyle, de préférence tert.-butyloxycarbonyle.

14. Procédé selon l'une des revendications 1 à 13, **caractérisé en ce que** le catalyseur de déshydrogénation [dans l'étape (B)] est choisi parmi les composés (sels et complexes) de fer, ruthénium et osmium ; cobalt, rhodium et iridium ; nickel, palladium et platine ; cuivre, argent et or, de préférence de composés sur la base de rhodium, palladium et platine.

15. Procédé selon la revendication 14, **caractérisé en ce que** le catalyseur de déshydrogénation représente un composé de palladium, de préférence un composé Pd(0), de préférence un complexe tris(dibenzylidenacétone)dipalladium-chloroforme ou un composé Pd(II), de préférence PdCl₂, Pd(dppe)₂, Pd(dppe)Cl₂, Pd(OAc)₂, Pd(dppe)(OAc)₂, des complexes π-allyle-Pd, de préférence un dimère π-allyle-Pd-chlorure.

16. Procédé selon l'une des revendications 1 à 15, **caractérisé en ce qu'**un agent complexant additionnel est ajouté pour la stabilisation thermique du complexe de palladium, de préférence 2,2'-bipyridyle ou 1,10-phénanthroline.

17. Procédé selon l'une des revendications 1 à 16, **caractérisé en ce qu'**une quinone substituée est utilisée en tant que quinone, de préférence une quinone substituée par alkyle C₁₋₄, halogène, cyano ou nitro.
